# EUROPÄISCHE PATENTSCHRIFT

(11) **EP 2 757 989 B1**
(45) Veröffentlichungstag und Bekanntmachung des Hinweises auf die Patenterteilung: **24.10.2018**
(21) Anmeldenummer: 12797723.9
(22) Anmeldetag: 17.09.2012
(51) Int. Cl.: A61B 17/88, A61B 17/86

(54) **CHIRURGISCHES INSTRUMENT**
SURGICAL INSTRUMENT
INSTRUMENT CHIRURGICAL

(30) Priorität: 22.09.2011 DE 102011053848; 08.06.2012 DE 102012104973
(43) Veröffentlichungstag der Anmeldung: 30.07.2014
(73) Patentinhaber: Combrowski, Zbiginiew, 78532 Tuttlingen (DE)
(72) Erfinder: Combrowski, Zbiginiew, 78532 Tuttlingen (DE)
(74) Vertreter: Patentanwälte und Rechtsanwalt Weiß, Arat & Partner mbB
(86) Internationale Anmeldenummer: PCT/EP2012/068249
(87) Internationale Veröffentlichungsnummer: WO 2013/041488

(56) Entgegenhaltungen:
- WO-A1-2010/142414
- WO-A2-02/45591
- DE-A1- 2 906 068
- FR-A1- 2 932 975

## Beschreibung

### Technisches Gebiet

Die Erfindung betrifft ein Chirurgisches Instrument nach dem Oberbegriff der Anspruchs 1.

### Stand der Technik

Zur Verbindung von Knochenteilen z.B. bei Osteotomien oder Traumata, werden verschiedene Schraubenimplantate auch mit Snap-Off-Schaft und in Verbindung mit Knochenplatten eingesetzt.

In diesem Zusammenhang wird auf die FR 2932975 A1 verwiesen, welcher einen Schraubendreher mit einer integrierten Messeinrichtung im Griff offenbart. Weiter wird auf die WO 2010/142414 A1 verwiesen, welche ein chirurgisches Instrument zur Einbringung einer Knochenschraube offenbart.
Daneben wird auf die DE 2906068 A1 hingewiesen, welche eine Einrichtung zur Befestigung von Knochenstücken offenbart. Ausserdem wird auf die WO 02/45591 A1 verwiesen, welche ein chirurgisches Element zur Anbringung von medizinischen Instrumenten oder zum Einbringen von Knochenschrauben geeignet ist.
Um die gewünschten Eigenschaften der Knochenverbindung zu bekommen, muss die Schraubenlänge und Schraubenposition der Schraube an die zu verbindenden Knochenteile angepasst werden.

Bei nicht kanülierten Schrauben geschieht das heutzutage mit z.B. Messinstrumenten wie Bohrtiefenmesser oder unter z.B. CT oder Röntgen, wobei zur Sicherheit immer mit einem im OP-Set befindlichen Messinstrument die Schraubenlänge bzw. die Bohrtiefe geprüft wird. Manche Hersteller bieten auch Instrumente zur manuellen Navigation der Schraubenposition im Set an.

Je nach Schraubenart, z.B. kanülierte Schrauben, wird zur Bestimmung der Schraubenlänge und zur sicheren Positionierung der Schraube auch ein Bohrdraht mit einer definierten Länge in gewünschter Tiefe in die Knochenteile eingebracht. Anhand der aus dem Knochen herausstehenden Länge des Bohrdrahtes können durch ein Längenmessinstrument die Bohrtiefe und somit auch die Schraubenlänge bestimmt werden.

Aus dem Stand der Technik sind eine Vielzahl von Vorrichtungen bekannt, die versuchen die Positionierung des Implantats zu erleichtern.

Beispielsweise offenbart die DE 20 2005 011 355 U1 ein Messinstrument zur Bestimmung der Tiefe und des Durchmessers einer Bohrung in einen Wirbel. Dazu wird ein Grössentaster, der in einem Tiefentaster verschieblich angeordnet ist, verwendet. Nachteiligerweise muss zusätzlich zu dem Implantierungswerkzeug auch der Tiefenmesser gelagert, durch die Wunde eingebracht und gereinigt werden.

Des Weiteren offenbart die EP 0 209 685 A2 eine Dübelhülse aus einem elastischen, gewebeverträglichen Kunststoff, die an ihrer Aussenfläche ein Gewinde aufweist. In Verbindung mit einem metallischen Schraubendreher kann die Position der Dübelhülse bei deren Montage am Röntgenschirm überwacht werden. Nachteiligerweise ist ebenfalls ein weiteres Instrument notwendig, um die Position der Schraube zu bestimmen.

Des Weiteren offenbart die DE 202 03 439 U1 einen chirurgischen Navigationsschraubenzieher mit einer Fixierhülse für Schrauben mit Schraubenkopfaussengewinde, wobei dieser eine integrierte Trackeraufsatzhülse für navigierte computerunterstützte Anwendungen aufweist. Dabei handelt sich um ein sehr aufwändiges Operationsset, das viele Einzelteile umfasst.

Hauptproblem aller am Markt befindlichen OP-Sets ist die Summe der verschiedenen Instrumente für herkömmliche OP-Methoden und für die computerassistierte Chirurgie sowie die Anzahl der einzelnen OP-Schritte.

Somit sind die Sets gross und für die einzelnen OPs abgestimmt und brauchen viel Platz zur Lagerung. Damit verbunden sind hohe Aufbereitungs- und Sterilisationskosten.

Sehr oft werden im Kleinfragmentbereich und in der Unfallchirurgie sehr viele OPs an einem Tag durchgeführt. So ist auch die Verfügbarkeit solcher Sets ein grosses Thema.

All den genannten Vorrichtungen ist der Nachteil gemeinsam, dass eine Fülle an unterschiedlichen Instrumenten und Vorrichtungen und Teilen für ein Operationsset benötigt wird.

Darüber hinaus werden Bohr-, Senk- und Fräs-Instrumente in der Chirurgie auch in kanülierter Ausführung aus verschiedenen, medizinisch zugelassenen Materialien hergestellt. Hierbei handelt es sich meistens um chirurgische Stähle. Dazu werden diese je nach Bedarf zusätzlich gehärtet oder vergütet.

Auch werden diese Instrumente für kombinierte Ausführungen, wie z.B. zum Bohren, Senken und/oder zum Gewindeschneiden in einem Instrument integriert um OP-Schritte zu sparen.

Je nach Einsatz sind die Instrumente sehr lang und müssen sehr biegsam sein, damit sie während des Einsatzes nicht brechen. Die Schneiden sind hart, meistens sogar zähhart. Aufgabe dieser harten Materialien ist es, durch alle bekannten Strukturen wie z.B. Knochen, Gewebe, Knorpel oder Zähne möglichst ohne Erwärmung beispielsweise zu bohren.

Je nach Operationsmethode werden die Instrumente z.B. in einer kanülierten Ausführung über einen Bohrdraht geführt eingesetzt. Hierzu werden die Instrumente mit einer durchgehenden Bohrung versehen, welche als Kanülierung bezeichnet wird.

Diese Kanülierungen sind bei diesen Instrumenten sehr schwer herstellbar, da diese Instrumente meistens sehr klein und/oder sehr lang sind. Die Materialien sind aufgrund ihrer Härte schwer zu bearbeiten. Hinzu kommt, dass das Bohren, Tiefenlochbohren sowie Schleifen sehr teure Herstellungsprozesse sind.

Ein grosser Nachteil dieser kanülierten Instrumente während der Operation ist ausserdem, dass beispielsweise beim Bohren Material in die lange Kanülierung gelangt und den Bohrdraht verklemmt und sich dann mitdreht. Der Bohrdraht wird somit tiefer eingebracht als gewollt, was zu Komplikationen während der Operation führen kann. Auch ist die Reinigung dieser Kanülierungen nach der Operation sehr schwierig und aufwendig.

Ein weiteres Ausführungsbeispiel aus dem Stand der Technik sind Bohrer ohne Kanülierung. Bei diesen werden am Aussenmantel bevorzugt zwei Spannuten bevorzugt spiralförmig angebracht. Diese haben die Aufgabe, das gebohrte Material aus dem Bohrloch abzuführen. Auch hier ist die Herstellung sehr teuer, weil diese Nuten zunächst gefräst und dann geschliffen werden müssen. Des Weiteren sind gerade kleine Bohrer durch die meistens zwei Spannuten sehr empfindlich und können leicht abbrechen.

### BESCHREIBUNG DER ERFINDUNG

Aufgabe der Erfindung ist es, ein chirurgisches Instrument zum Einbringen eines Implantats bereitzustellen, das sehr einfach ausgebildet ist und das die Ermittlung der Bohrtiefe, Navigation und einfache Reinigung ermöglicht. Weiterhin sollen teure Fertigungsprozesse vermieden werden.

Zur Lösung der Aufgabe führen die Merkmale des Anspruchs 1.

In typischen Ausführungsbeispielen umfasst ein chirurgisches Instrument zum Einbringen eines Implantats einen Schaft.

Vorzugsweise ist der Schaft geeignet an seinem proximalen Ende ein Implantat, insbesondere eine Schraube mit einer Kanülierung aufzunehmen. Vorzugsweise weist der Schaft bevorzugt an einem Ende eine stückweise Kanülierung auf. Dadurch ergibt sich der Vorteil, dass ein Bohrdraht oder ein sog. K-Draht in dem Schaft geführt werden kann.

Die Kanulierung ist dahinter weitestgehend offen, bevorzugt einseitig oder mehrseitig geschlitzt, oder ganz geschlitzt. Das Ziel ist, die Festigkeit der Instrumente nicht zu verschlechtern und die Reinigbarkeit und Sterilisierbarkeit zu verbessern. Der geschlitzte Aussenmantel soll gleichzeitig mehr Platz zur z.B. Skalierung, Beschriftung der Instrumente bieten. Bevorzugt soll mindestens ein Teil kanuliert und/oder mindestens ein Teil teilweise geschlitzt sein. In welchem Verhältnis, Abständen und oder Winkel ist je nach Anwendung und Instrument unterschiedlich. Noch bevorzugter soll mindestens ein Teil kanuliert und mindestens ein Teil von einer oder mehreren Seiten geschlitzt sein um eine optimale Führung des z.B. Bohrdrahtes zu gewährleisten. In einem weiteren bevorzugten Ausführungsbeispiel sollen die Schlitze von mehreren Seiten so angebracht sein, dass diese sich in der nahezu Mitte, bevorzugt axial verbinden und so eine Kanülierung für z.B. einen Bohrdraht entsteht. Auch kann die Kanulierung weitere Aufgaben übernehmen wie z.B. Kanal zum Einführen einer Sonde oder eines Ankers oder anderer Instrumente.

Zweckmässigerweise umfasst das chirurgische Instrument einen Griff, der mit dem Schaft an einem distalen Ende des Schafts verbunden ist. Vorzugsweise ist der Schaft mit dem Griff fest verbunden.

In alternativen Ausführungsbeispielen ist der Griff mit dem Schaft lösbar und wechselbar verbunden. Vorzugsweise weist der Griff ein Kupplungselement, insbesondere eine Steckkupplung, ein Gewinde oder ein Rastelement auf, das mit einem geeigneten Gegenstand an einem Ende des Schafts lösbar verbunden werden kann.

In typischen Ausführungsbeispielen weist der Schaft einen Schlitz auf. Bevorzugt ist der Schlitz geeignet die Kanülierung des Schafts mit einer Oberfläche des Schafts zu verbinden. Dadurch ergibt sich der Vorteil, dass die Kanülierung bzw. der Schlitz leichter herstellbar ist. Des weiteren ist vorteilhaft, dass der Schaft und die Kanülierung durch den offenen Schlitz leicht zu reinigen sind.

Ein weiterer Vorteil ist, dass ein in der Kanülierung geführter K-Draht bzw. dessen Ende beobachtet werden kann. Manchmal passiert es, dass der K-Draht unerwünschter Weise beim Einbringen des Implantats bzw. Eindrehen der Schraube mit vorgetrieben wird. Durch die geschlitzte Kanülierung ist für den Arzt Sichtkontrolle zur Lage des Bohrdraht oder K-Draht möglich.

In typischen Ausführungsbeispielen ist der Schlitz einseitig ausgeführt. Bevorzugt ist der Schlitz im wesentlichen über eine gesamte Länge der Kanülierung ausgeführt.

In alternativen Ausführungsbeispielen weist der Schaft eine Mehrzahl von Schlitzen auf. Dadurch ergibt sich der Vorteil, dass die Kanülierung noch besser gereinigt werden kann.

In typischen Ausführungsbeispielen umfasst ein chirurgisches Instrument zum Einbringen eines Implantats einen Schaft, wobei der Schaft geeignet ist, an seinem proximalen Ende das Implantat, insbesondere eine Schraube aufzunehmen. Vorzugsweise handelt es sich um einen massiven Schaft, der nicht kanüliert ist und keinen Schlitz aufweist.

Zweckmässigerweise weist das chirurgische Instrument einen Messbügel auf. Vorzugsweise ist der Messbügel geeignet die Bohrtiefe und/oder Implantatlänge, insbesondere die Schraubenlänge, zumindest näherungsweise zu bestimmen.

In typischen Ausführungsbeispielen ist der Messbügel verschieblich mit dem Schaft des chirurgischen Instruments verbunden werden. In typischen Ausführungsbeispielen weist der Messbügel einen Haken auf. Vorzugsweise wird der Haken an den Schaft geclipst. Vorzugsweise ist der Messbügel lösbar mit dem Schaft verbunden. Dadurch ergibt sich der Vorteil, dass der Messbügel mit unterschiedlichen Schäften kombiniert werden kann.

In typischen Ausführungsbeispielen weist der Schaft eine Skalierung auf. Vorzugsweise ist die Skalierung geeignet, eine Implantatlänge und/oder eine Einbringtiefe zu bestimmen. Vorzugsweise eignet sich die Skalierung in Zusammenwirken mit einem anderen Element zur Bestimmung der Einbringtiefe, bzw. der Implantatlänge.

In typischen Ausführungsbeispielen ist das andere Element ein hinteres Ende des K-Drahts oder des Bohrdrahts, der in der Kanülierung geführt ist, und durch den Schlitz gesehen werden kann.

In typischen Ausführungsbeispielen ist das andere Element der Messbügel und/oder eine Anzeige des Messbügels, die auf der Skalierung eine Implantatslänge und/oder eine Einbringtiefe anzeigt.

In typischen Ausführungsbeispielen ist der Schaft an seinem proximalen Ende über eine Sollbruchstelle mit einem Implantat, insbesondere einer Schraube verbunden. Vorzugsweise handelt es sich bei dem Implantat um ein sog. genannte Snap-off-Implantat. Vorzugsweise bricht das Implantat bei erreichen eines gewissen Drehmoments an der Sollbruchstelle ab. Vorzugsweise wird nach dem Abbrechen des Implantats an der Sollbruchstelle an dem proximalen Ende des Schafts ein Mitnehmer freigelegt. Vorzugsweise eignet sich der Mitnehmer zum Eingriff in ein entsprechendes Gegenstück an dem Implantat, um das Implantat ggf. noch weiter in den Knochen einzudrehen. Dadurch ergibt sich der Vorteil, dass das Implantat beim Einbringen nicht verloren werden kann, und dass der Operateur den kompletten Einbringvorgang, sowie das Bestimmen der Schraubenlänge mit einem Instrument ausführen kann.

In typischen Ausführungsbeispielen weist der Schaft an seinem proximalen Ende einen Mitnehmer, geeignet mit dem Implantat in Eingriff gebracht zu werden. In typischen Ausführungsbeispielen handelt es sich bei dem Mitnehmer um eine Schraubendreherklinge. Vorzugsweise ist der Mitnehmer als Schlitz, Torx, Torq, Kreuz, Kreuzschlitz, Sechskant oder Inbus ausgebildet. Zweckmässigerweise wird der Mitnehmer erst nach dem Abbrechen des Implantats an der Sollbruchstelle freigelegt.

In typischen Ausführungsbeispielen weist der Schaft an seinem proximalen Ende eine Kupplung auf. Vorzugsweise ist die Kupplung geeignet zur Verbindung mit einem auswechselbaren Mitnehmer. Vorzugsweise handelt es sich bei dem Mitnehmer um eine Schraubendreherklinge. Vorzugsweise weist die Schraubendreherklingen einen Schlitz, Torx, Torq, Kreuz, Kreuzschlitz, Sechskant oder Inbus auf.

Vorzugsweise ist der auswechselbare Mitnehmer in der Art eines Bits oder einer Nuss ausgebildet. Vorzugsweise werden die Implantate, insbesondere Schauben einzeln steril verpackt, zur OP bereit gestellt.

In typischen Ausführungsbeispielen weist der Messbügel einen Anschlag oder eine Spitze zum Anlegen eines Implantatendes am Knochen, an der Haut oder dem Gewebe und/oder eines Schaftendes auf. Vorzugsweise gibt es Messbügel in zwei Ausführungen. Dabei handelt es sich um eine extrakorporale und eine intrakorporale Ausführung. Die extrakorporale Ausführung des Messbügels dient dabei dem Messen an dünnen Hautschichten von aussen und die intrakorporale Ausführung dient direkt dem Messen durch den Zugang bevorzugt am Knochen.

Vorzugsweise umfasst der Messbügel eine weitere Spitze, die es dem Arzt ermöglicht beim An- oder Eindrücken der Spitze durch Haut und Gewebe an eine Knochenseite und durch Andrücken der Schraubendreherspitze auf der anderen Seite des Knochens die Schraubenlänge und Einbringrichtung zu bestimmen. Das gleiche ist auch statt mit dem Schraubendreher beispielsweise mit einem Bohrer möglich. Wenn am Aussenmantel des Bohrers eine Skalierung angebracht ist, ist es vorteilhaft möglich ein navigiertes Bohren oder ein Bohren auf eine bestimmte Tiefe zu ermöglichen.

In typischen Ausführungsbeispielen eignet sich der Bügel mit der Bügelspitze als Navigator zur Findung der richten Bohr- oder Schraubenposition. Vorteilhafterweise eignet sich der Messbügel auch als Fixierung und Gegenhalter beim Einschrauben von Hand oder mit einer Maschine bzw. einem Robroter oder Manipulator. Der Messbügel kann auch als Anschlag für eine definierte maximale Bohrtiefe dienen.

In typischen Ausführungsbeispielen weist der Messbügel einen Anschlag vorzugsweise Spitze zum Anlegen eines Implantatendes am Knochen, Haut oder Gewebe und/oder eines Schaftendes auf.

Vorzugsweise gibt es die Messbügel in zwei Ausführungen Extra- und Intrakorporal. Der Extrakorporale zum an dünnen Hautschichten von aussen messen der Intrakorporale um direkt durch den Zugang am Knochen zu messen.

Vorzugsweise umfasst der Messbügel eine weitere Spitze, die es dem Arzt ermöglicht beim An- oder Eindrücken der Spitze durch an / in der Haut, Knochen oder und Gewebe an eine Knochenseite und durch Andrücken der Schraubendreherspitze auf der anderen Seite des Knochens die Schraubenlänge und Einbringrichtung zu bestimmen.

Das gleiche ist auch statt dem Schraubendreher mit beispielsweise einem Bohrer möglich. Wenn am Bohrer Aussenmantel beispielsweise eine Skala angebracht ist, Vorteil ist auch hier das navigierte Bohren, sowie das Bohren auf eine bestimmte Tiefe.

In typischen Ausführungsbeispielen eignet sich der Bügel mit der Bügelspitze als Navigator zur Findung der richtigen Bohr- oder Schraubenposition. Vorteilhafterweise eignet sich der Messbügel auch als Fixierung und Gegenhalter beim Einschrauben von Hand oder mit einer Maschine bzw. einem Roboter oder Manipulator. Der Bügel kann auch als Anschlag für z.B. eine definierte maximale Bohrtiefe dienen.

Vorzugsweise kann der Messbügel auch in Verbindung mit chirurgischen Instrumenten, die einen kanülierten und geschlitzten Schaft aufweisen, eingesetzt werden. Vorteilhafterweise kann dadurch die Gewebedicke, die durchdrungen werden muss, und die Einschraubtiefe in den Knochen mit einem Instrument ermittelt werden .

In typischen Ausführungsbeispielen umfasst das erfindungsgemässe chirurgische Instrument einen aktiven oder passiven Tracker. Vorzugsweise ist der Tracker am Schraubendreherschaft oder -Griff angebracht. Vorzugsweise kann dabei die Schraubenposition bei minimal invasiven oder computerassistierten OP's überwacht werden. Vorzugsweise ist der Tracker einstückig mit dem chirurgischen Instrument verbunden. In typischen Ausführungsbeispielen ist der Tracker ausgebildet, um lösbar am Schraubendreherschaft oder -Griff befestigt zu werden.

In typischen Ausführungsbeispielen weist der Schaft eine Schraubendreherklinge oder einen Mitnehmer in Kreuzschlitz, Hexagon, Torx oder Ankeraufnahmesystem auf.

Vorzugsweise sind alle Schraubendreherklingen so ausgebildet, dass sie mit dem Schaft eine Einheit bilden und während der OP nicht verloren gehen können. Zweckmässigerweise bilden der Schaft, der Mitnehmer und das Implantat einen starren Körper (rigid body) und sind unverlierbar, um ein computerunterstütztes, präzises und sicheres Arbeiten zu ermöglichen.

In typischen Ausführungsbeispielen umfasst der Schaft einen Doppelmitnehmer. Vorzugsweise eignet sich der Doppelnehmer zur Verbindung einer unverlierbaren Snap-Off-Schraube oder eines unverlierbaren Snap-Off-Implantats mit dem Schaft.

In typischen Ausführungsbeispielen umfasst der Schaft ein Schnellwechselsystem, insbesondere ein Bajonette-System zum Verbinden mit einem auswechselbaren Mitnehmer.

In typischen Ausführungsbeispielen weist der Schaft einen Mitnehmer auf, geeignet zum Einbringen von Ankern, Snap-Off-Anker-Systemen, Staples oder Snap-Off-Staples. Dadurch ergibt sich der Vorteil, dass das chirurgische Instrument zum Einbringen von einer Vielzahl von Implantaten geeignet ist.

In typischen Ausführungsbeispielen umfasst das chirurgische Instrument einen Drehmomentbegrenzer. Vorzugsweise ist der Drehmomentbegrenzer als Drehmomentpatrone ausgebildet. In typischen Ausführungsbeispielen kann der Drehmomentbegrenzer dem chirurgischen Instrument bei Bedarf hinzugefügt werden.

In typischen Ausführungsbeispielen weist das chirurgische Instrument eine Ratsche auf. Dadurch ergibt sich der Vorteil, dass das chirurgische Instrument einfacher bedient werden kann. In typischen Ausführungsbeispielen umfasst die Ratsche einen Drehmomentbegrenzer.

In typischen Ausführungsbeispielen weist der Schaft eine Schutzhülse auf. Dadurch ergibt sich der Vorteil, dass das chirurgische Instrument bzw. das Implantat weichteil- und gewebeschonend eingebracht werden kann. Zweckmässigerweise ist die Hülse federnd ausgebildet. Zweckmässigerweise ist die Hülse auswechselbar. Vorzugsweise ist die Hülse konisch ausgebildet.

In typischen Ausführungsbeispielen ist die Hülse geschlitzt ausgebildet. Dadurch ergibt sich der Vorteil, dass beispielsweise bei Kontakt mit dem Knochen die Hülse durch die Schraube oder durch eine Mechanik aufgeweitet werden kann.

In typischen Ausführungsbeispielen weist der Messbügel eine integrierte Haltevorrichtung für Knochenfragmente, für Bohrhülsen und/oder eine Schablonenhalterung als Zielgerät zur Revision von Schrauben auf. Vorzugsweise weist der Messbügel eines Haltevorrichtung zur Fixierung von Platten auf.

In typischen Ausführungsbeispielen weist der Schaft und/oder der Schraubendrehergriff eine Aufnahme für eine Schnellwechselaufnahme auf. Dadurch ergibt sich der Vorteil, dass das chirurgische Instrument in einen OP-Roboterarm eingespannt werden kann. Dadurch ergibt sich der Vorteil, dass das chirurgische Instrument auch in roboterunterstützen Operationen verwendet werden kann.

In typischen Ausführungsbeispielen weist der Messbügel eine Markierung auf. Bevorzugt eignet sich die Markierung zur Anzeige der gemessenen Implantatslänge auf der Skalierung des Schafts.

Gesondert wird Schutz beansprucht für ein chirurgisches Instrument umfassend einen Schaft mit einer Kanülierung und einem Schlitz und einem Messbügel. Dadurch ergibt sich der Vorteil, dass durch den Schlitz das Ende eines Bohrdrahts beobachtet werden kann und mit dem Messbügel eine überprüfende oder weitere Vergleichsmessung durchgeführt werden kann. Insbesondere eignet sich der Messbügel zur Bestimmung der zu durchdringenden Gewebedicke sowie der Navigation.

Gesondert wird Schutz beansprucht für einen Schaft für ein chirurgisches Instrument mit den beschriebenen Merkmalen.

Vorzugsweise wird auf Implantatmagazine in den Instrumentensets aufgrund der besseren Verfügbarkeit und Aufbereitungseinsparung verzichtet.

In typischen Ausführungsbeispielen wird auf Standardinstrumente im Set verzichtet. Normalerweise sind diese in ausreichenden Mengen in Krankenhäusern vorrätig.

Vorzugsweise befinden sich nur OP spezifische Instrumente im Set, die einzeln oder steril verpackt, bereit gestellt werden. Dadurch ergibt sich der Vorteil, dass die erfindungsgemässen chirurgischen Instrumente als Mehrweg- oder auch als Einweginstrumente ausgebildet sein können.

Dadurch, dass Schaft und Griff lösbar miteinander verbunden sind, ergibt sich die Möglichkeit, dass die erfindungsgemässen Schäfte schnell mit anderen Aufnahmen verbunden werden können. Dadurch ergibt sich der Vorteil, dass durch Zusatzkomponenten der Einsatz in Schnellwechsel- oder Koppelsystemen möglich ist, wodurch ein schneller Wechsel von manueller oder computernavigierter Operation möglich ist. Dadurch wird die Prozesssicherheit erhöht.

Ein weiterer Vorteil ist, dass mit dem erfindungsgemässen chirurgischen Instrument manuelle und computergestützte OP-Methoden realisiert werden können. Des weiteren wird vorteilhafterweise die Anzahl der Instrumente minimiert wird, und die Anzahl der OP-Schritte, durch die Verwendung eines einzigen Instruments, verringert. Dadurch wird die Operation vereinfacht.

Ein weiterer Vorteil der erfindungsgemässen chirurgischen Instrumente ist, dass diese neben einer Mess- auch eine Vergleichsfunktion und/oder Navigationsfunktion besitzen. Dadurch kann auf die Verwendung zusätzlicher Messinstrumente verzichtet werden.

Bevorzugt soll z.B. bei der Herstellung von nicht kanülierten Bohrern oder Stufenbohrern auf Spiralnuten am Aussenmantel verzichtet werden. Diese sollen nur teilweise geschlitzt werden, was zu einer günstigeren Herstellung bei besserer Qualität führt.

Noch bevorzugter sollen diese Bohrinstrumente die von mehreren Seiten angebrachten Schlitze sich an mindestens einer Stelle verbinden, noch besser durchbrechen, so kann z.B. beim Bohren das Material von der zweiten Schneide auch in den Schlitz gelangen. Vorteil ist hier, dass das Material im Schlitz schonend gesammelt wird und aus diesem einfach entnommen werden kann, um beispielsweise irgendwo anders eingesetzt zu werden, oder als Probeentnahme zu dienen.

Bevorzugt soll diese Technik bei Schraub-, Einschlag-, Bohr-, Senk-, Fräsinstrumenten, Instrumentenschäften oder Führungshülsen in der Chirurgie eingesetzt werden, aber auch z.B. bei Führungsrohren in der Endoskopie.

Grundsätzlich soll auch die Erfindungsidee Verwendung bei Kombinationen verschiedener Instrumente finden. So sind beispielsweise kombinierte Bohr- und Senkinstrumente auch in kanulierter Ausführung denkbar. Diese Instrumente sollen hierbei einteilig oder mehrteilig zerlegbar sein. Alle Ausführungen können entlang der Schlitze an dem Aussenmantel grosszügig mit Markierungen / Skalen und / oder Beschriftungen versehen werden, wobei diese Skalen und / oder Markierungen und oder Beschriftungen und oder Farbkodierungen kombiniert beispielsweise zur Bestimmung der Schraubenlänge und/oder der Tiefe des in den Knochen eingebrachten Bohrdrahtes oder Bohrers dienen können.

Die erfindungsgemässen Instrumente können auch z.B. mit Schnellrastsystemen ausgestattet sein. Diese dienen beispielsweise der zur einfachen Einstellung der Schraubenlänge. Alle Ausführungen dienen einzeln oder auch in Verbindung mit z.B. einer Bohrlehre, eines Messbügels, eines Navigators oder einer Kombination dieser, zur besseren Positionierung, Führung, als Gegenhalt oder Anschlag. Alle erfindungsgemässen Ausführungen können auch als Einweginstrumente ausgebildet sein, was aufgrund der kostengünstigen Herstellung möglich wird. Alle Ausführungen können auch als Hybrid aus z.B. zwei Werkstoffen hergestellt werden. Hierbei kommt es auf die vom Nutzer geforderten Voraussetzungen an Preis und Gebräuchlichkeit an. Die erfindungsgemässen Ausführungen sind derart ausgeführt, dass der Schaft und der Griff fest miteinander verbunden sein können oder beispielsweise der Griff und der Schaft mit einer Kupplung verbindbar sind.
Die erfindungsgemässen Ausführungen mit Schaft und Kupplung für Bits oder andere Einsätze sind derart gestaltet, dass die Bits z.B. als Schraub-, Bohrbit oder Träger für z.B. Anker dienen können.
Ein grosser Vorteil ist die günstige, einfache Herstellung, was gerade bei Einweginstrumenten sehr wichtig ist. Aber auch bei Mehrweginstrumenten sind diese mindestens teilweise geschlitzte Ausführung in der Fertigung wie auch nach dem Operationseinsatz einfacher, schneller zu reinigen und zu Sterilisieren und somit besser reproduzierbar und entsprechen eher heutigen Hygienevorschriften zur Erreichung validierter Reinigungs- und Sterilisationsprozesse in der Medizintechnik.

Ziel ist auch diese mindestens teilweise oder ganz geschlitzten Instrumente nicht nur für die genannten Instrumente zu begrenzen, sondern auch bei anderen Instrumenten die traditionell eine Kanulierung besitzen anzuwenden.

Denkbar ist auch diese mindestens teilweise geschlitzte Ausführung auch bei handelsüblichen Werkzeugen einzusetzen.

### KURZE BESCHREIBUNG DER ZEICHNUNGEN

Nachfolgend wird die Erfindung anhand der beiliegenden Figuren kurz beschrieben, wobei die Figuren zeigen:
**Figur 1** zeigt eine schematische Darstellung einer Seitenansicht eines erfindungsgemässen chirurgischen Instruments mit einem geschlitzten Schaft und einer Knochenschraube als Implantat, die über eine Sollbruchstelle mit dem Schaft verbunden ist;
**Figur 2** zeigt eine schematische Darstellung eines Schnitts eines des Ausführungsbeispiels nach Figur 1 mit einem K-Draht;
**Figur 3** zeigt eine schematische Darstellung einer perspektivischen Ansicht eines kanülierten geschlitzten Schafts des chirurgischen Instruments des Ausführungsbeispiels der Figuren 2 und 3;
**Figur 4** zeigt eine schematische Darstellung einer Seitenansicht eines weiteren Ausführungsbeispiels des erfindungsgemässen chirurgischen Instruments mit einem kanülierte geschlitzten Schaft und einem auswechselbaren Mitnehmer mit einer Knochenschraube;
**Figur 5** zeigt eine schematische Darstellung eines Schnitts des Ausführungsbeispiels des erfindungsgemässen chirurgischen Instruments nach Figur 4 mit einem kanülierte geschlitzten Schaft und einem auswechselbaren Mitnehmer mit einem K-Draht;
**Figur 6** zeigt eine schematische Darstellung einer Seitenansicht eines weiteren Ausführungsbeispiels eines erfindungsgemässen chirurgischen Instruments mit einem Messbügel;
**Figur 7** zeigt einen vergrösserten Ausschnitt des Messbügels und des Schafts nach Figur 6;
**Figur 8** zeigt eine weitere schematische Darstellung eines vergrösserten Ausschnitts des Messbügels und des Schafts nach Figur 6;
**Figur 9** zeigt eine schematische Darstellung des erfindungsgemässen chirurgischen Instruments mit einem Messbügel und einer Knochenschraube als Implantat;
**Figur 10** zeigt eine schematische Darstellung eines vergrösserten Ausschnitts der Schraube, des Messbügels und des Schafts nach Figur 9;
**Figur 11** zeigt eine schematische Darstellung eines weiteren vergrösserten Ausschnitts des Schafts und des Messbügels nach Figur 9;
**Figur 12** zeigt eine schematische Darstellung einer perspektivischen Ansicht des Messbügels der Figuren 6 und 9.
**Figur 13** zeigt eine teilweise geschnittene Seitenansicht eines weiteren einteilig ausgebildeten Ausführungsbeispiels eines erfindungsgemässen Instruments;
**Figur 14** zeigt eine perspektivische Ansicht des Instruments nach Figur 13;
**Figur 15** zeigt eine teilweise geschnittene Seitenansicht eines weiteren einteilig ausgebildeten Ausführungsbeispiels eines erfindungsgemässen Instruments;
**Figur 16** eine perspektivische Ansicht des Instrumentes nach Figur 15;
**Figur 17** zeigt eine vergrösserte Ansicht einer Spitze des Instrumentes nach Figur 15 und 16;
**Figur 18** zeigt eine Draufsicht auf ein weiteres Ausführungsbeispiels eines erfindungsgemässen Instrumentes in mehrteiliger Ausführung;
**Figur 19** zeigt eine teilweise geschnittene Seitenansicht des Instruments nach Figur 18;
**Figur 20** zeigt eine perspektivische Ansicht des Instrumentes nach Figur 18;
**Figur 21** zeigt eine Explosionsdarstellung des Instrumentes nach Figur 20;
**Figur 22** zeigt eine perspektivische Ansicht eines weiteren einteilig ausgebildeten Ausführungsbeispiels eines Instrumentes; und
**Figur 23** zeigt eine vergrösserte Ansicht einer Spitze des Instrumentes nach Figur 22.

### AUSFÜHRUNGSBEISPIELE

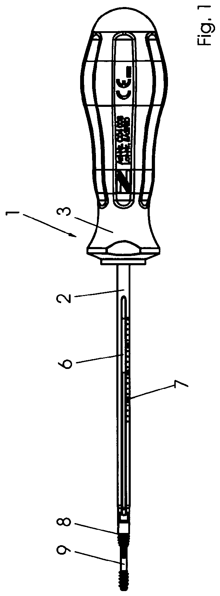

Figur 1 zeigt als erfindungsgemässes chirurgisches Instrument 1 einen Schraubendreher mit einem kanülierten Schaft 2 und einem Griff 3.

Der kanülierte Schaft 2 und der Griff 3 können entweder fest verbunden oder, beispielsweise über eine Kupplung auswechselbar verbunden sein.

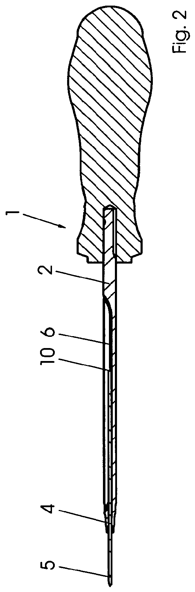

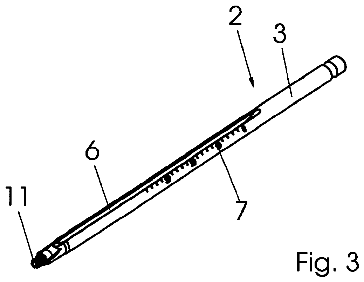

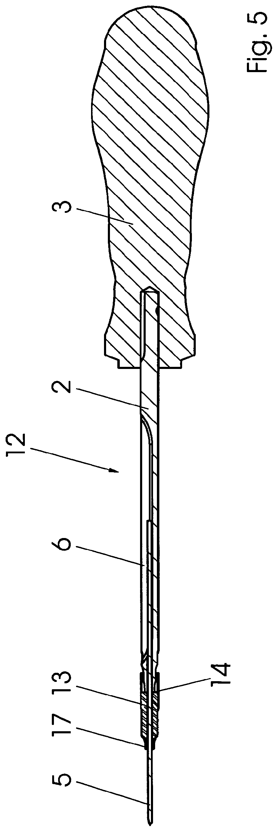

Der kanülierte Schaft 2 ist auch in den Figuren 2, 3, 4 und 5 dargestellt. Wie in dem Schnittdarstellungen der Figuren 2 und 5 erkennbar, weist der Schaft 2 eine Kanülierung 4 auf. Die Kanülierung 4 ist geeignet zur Aufnahme eines in den Figuren 2 und 5 dargestellten K-Drahts 5 (so genannter Kirschner Draht oder Bohrdraht). Der kanülierte Schaft 2, weist wie in den Figuren 1 bis 5 dargestellt, einen Schlitz 6 auf.

Der Schlitz 6 verbindet die Kanülierung 4 mit einer äusseren Oberfläche des Schafts 2.

Zur Herstellungsweise des Schafts 2 wird angemerkt, dass bei diesen wahlweise zuerst die Kanülierung 4 eingebracht wird und der Schaft 2 dann bis zur Kanülierung aufgeschlitzt wird. Des Weiteren ist auch eine Herstellungsweise denkbar, bei der ein massiver Schaft 2 geschlitzt wird und anschliessend in einem vorderen Bereich, zur Führung des K-Draht 5 wieder verschlossen wird. Der Vorteil der zweiten Herstellungsweise ist, dass auf das Einbringen einer Längsbohrung in den Schaft 2 verzichtet werden kann.

Des Weiteren weist der Schaft 2, wie in den Figuren 1, 3 und 4 dargestellt, eine Skalierung 7 auf. Die Skalierung 7 ist parallel zum Schlitz 6 angeordnet.

Bei dem Ausführungsbeispiels des chirurgischen Instruments 1 nach der Figur 1 ist der Schaft über eine Sollbruchstelle 8 mit einer Knochenschraube 9 als Implantat verbunden.

Die Funktion des chirurgischen Instruments 1 nach dem Ausführungsbeispiel der Figuren 1, 2 und 3 ist folgende:
In den Schaft 2 wird ein K-Draht 5 eingebracht. Über die Lage eines oberen Endes 10 des K-Drahts 5, wie in Figur 2 dargestellt, wird an der Skalierung 7 des Schafts 2 eine Schraubenlänge der Knochenschraube 9 und damit die Einbringtiefe angezeigt.

Die Knochenschraube 9 wird mit dem chirurgischen Instrument 1 in einen Knochen eingebracht, bis ein gewisses Drehmoment erreicht wird und die Knochenschraube 9 an der Sollbruchstelle 8 von dem Schaft 2 des chirurgischen Instruments 1 abbricht. Nach dem Funktionsprinzip einer Snap-Off-Schraube wird bei der Knochenschraube 9 nach dem Abbrechen in den Schraubenkopf ein Mitnehmer frei gelegt. Falls notwendig, kann mit einem entsprechenden Mitnehmer 11 am proximalen Ende des Schafts 2 in den Mitnehmer im Schraubenkopf der Knochenschraube 9 gegriffen werden und die Knochenschraube noch weiter eingedreht werden.

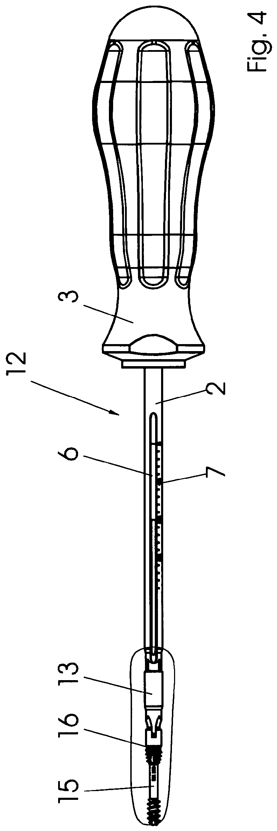

Die Figuren 4 und 5 zeigen ein weiteren Ausführungsbeispiel eines chirurgischen Instruments 12. Das chirurgische Instrument ist im Wesentlichen analog zu dem chirurgischen Instrument 1 ausgebildet und umfasst ebenfalls einen kanülierten Schaft 2 und einen Griff 3.

Im Gegensatz zum Ausführungsbeispiel des chirurgischen Instruments 1 weist das chirurgische Instrument 12 einen auswechselbaren Mitnehmer 13 auf. Der auswechselbare Mitnehmer 13 kann nach der Funktionsweise eines Bits mit einem proximalen Ende 14 des Schafts 2 auswechselbar verbunden werden. Dazu ist das proximale Ende 14 des Schafts 2 vorzugsweise als Kupplungselement ausgebildet, das mit dem auswechselbaren Mitnehmer 13 nach Art eines Bits oder einer Nuss in Wirkverbindung gebracht werden kann.

Wie in Figur 4 dargestellt, ist der auswechselbare Mitnehmer 13 vorzugsweise über eine Sollbruchstelle 16 mit der Knochenschraube 15 verbunden.

Die Funktionsweise ist im Wesentlichen analog zu der des vorhergehend beschriebenen Ausführungsbeispiels.

Für den Operateur ergibt sich bei dem chirurgischen Instrument 12 der Vorteil, das er einen gewohnten Griff 3 und einen gewohnten Schaft 2 zur Verfügung hat, den er auf einfache Art und Weise mit einer Knochenschraube 15, die über die Sollbruchstelle 16 mit dem Mitnehmer 13 verbunden ist, kombinieren kann. Dadurch ergibt sich die Möglichkeit, dass ohne weiteres unterschiedliche Knochenschrauben bzw. wechselbare Mitnehmer 13 mit dem Schaft 2 und dem Griff 3 kombiniert werden können.

Analog zum Ausführungsbeispiel des chirurgischen Instruments 1 kann die Knochenschraube 15 nach dem Abscheren an der Sollbruchstelle 16 mit einem Mitnehmer 17 des auswechselbaren Mitnehmers 13 weiter in den Knochen eingebracht werden. Der Mitnehmer 17 des auswechselbaren Mitnehmers 13 wird ebenfalls erst nach dem Abbrechen der Knochenschraube 15 an der Sollbruchstelle 16 freigelegt.

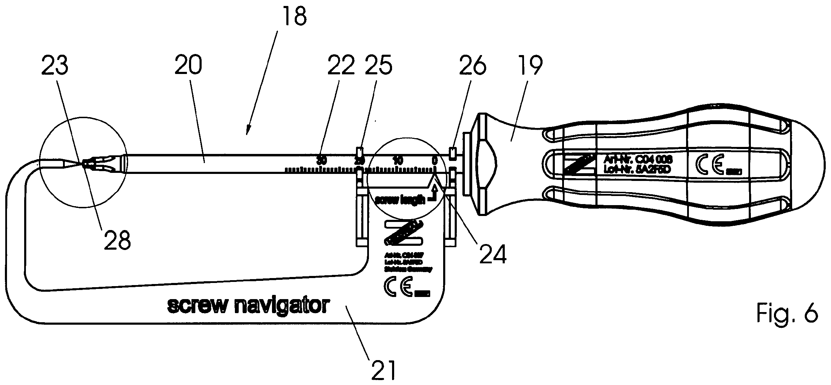

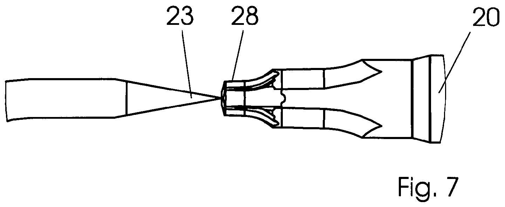

Die Figuren 6 bis 12 offenbaren ein weiteres Ausführungsbeispiel eines chirurgischen Instruments 18. Das chirurgische Instrument umfasst einen Griff 19 und einen Schaft 20. Der Schaft 20 weist in dem dargestellten Ausführungsbeispiel keine Kanülierung und keinen Schlitz auf.

In weiteren nicht dargestellten Ausführungsbeispielen umfasst ein chirurgisches Instrument, wie das chirurgische Instrument 18 einen Schaft, der eine Kanülierung und einen Schlitz aufweist, analog zum Schaft 2 der Figuren 1 bis 5.

Ein nicht dargestelltes Ausführungsbeispiel eines erfindungsgemässen chirurgischen Instruments umfasst einen Griff 19 einen Messbügel 21 und einen Schaft 2 nach den Figuren 1 - 5.

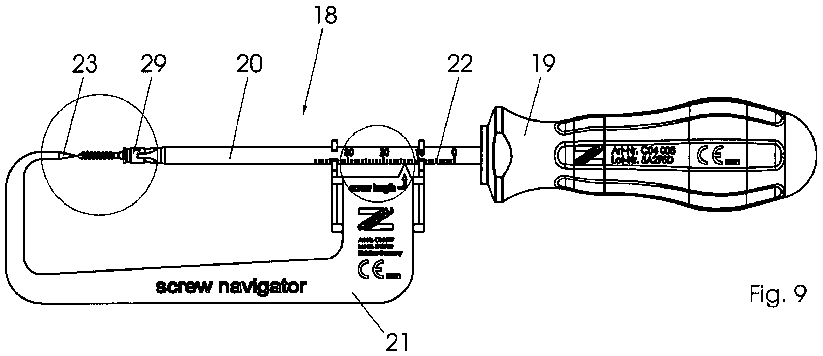

Des Weiteren umfasst das chirurgische Instrument 18 der Figuren 6 und 9 einen Messbügel 21. Der Schaft 20 weist an seiner Oberseite eine Skalierung 22 auf.

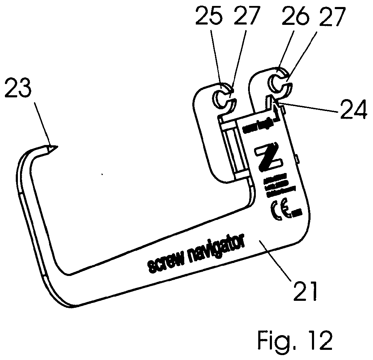

Der Messbügel 21 umfasst, wie in Figur 12 dargestellt, eine Messspitze 23 und eine Anzeige 24.

Des Weiteren umfasst der Messbügel 21, zum verschieblichen Festlegen des Messbügels 21, an dem Schaft 20 oder 2 einen ersten Haken 25 und einen zweiten Haken 26. Die Haken 25 und 26 weisen jeweils eine Öffnung 27 auf. Vorzugsweise nimmt die Öffnung 27 jeweils nur einen Winkelbereich zwischen 90° und 5° ein. Zweckmässigerweise können die Haken 25 und 26 über den Schaft 20 bzw. 2 geclipst werden.

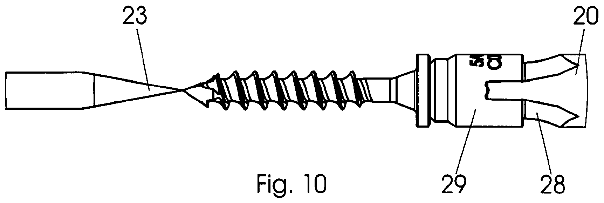

Der Schaft 20 weist einen Mitnehmer 28, wie in Figur 6 dargestellt, auf. Der Mitnehmer eignet sich zur Aufnahme einer Knochenschraube 29, wie in den Figuren 9 und 10 dargestellt.

Grundsätzlich kann der ungeschlitzte und unkanülierte Schaft 20 auch mit einem proximalen Ende analog zum Schaft 2 nach den Figuren 1, 2, 3, 4 und 5 ausgebildet sein. Dass heisst, ein ungeschlitzter und unkanülierter Schaft könnte ebenfalls über eine Sollbruchstelle mit einer Knochenschraube verbunden sein, oder eine Kupplung aufweisen, die sich zur Verbindung mit einem auswechselbaren Mitnehmer eignet.

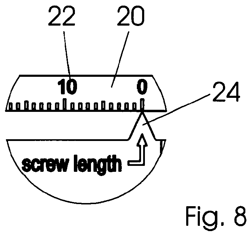

Die Funktionsweise des chirurgischen Instruments 18 nach den Figuren 6 und 9 ist folgende:
Der Messbügel 21 wird über die Haken 25 und 26 an den Schaft 20 montiert. Dabei berührt eine Spitze 23 des Messgeräts 21 ein vorderes Ende des Mitnehmers 28 des Schafts 20. Über die Anzeige 24 des Messgeräts 21 wird ein Nullpunkt auf der Skalierung 22 des Schafts festgelegt bzw. bestimmt, wie in Figur 8 dargestellt.
In weiteren Ausführungen können hier auch Korrekturwerte berücksichtigt sein.

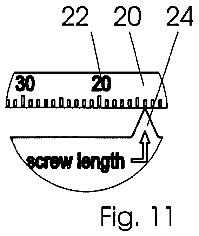

Nach Aufnahme der Knochenschraube 9 mit dem Mitnehmer 28 des Schafts 20, wie in den Figuren 9 und 10 dargestellt, berührt die Spitze 23 eine Spitze der Knochenschraube 29. Dadurch wird der Messbügel 21 so am Schaft 20 verschoben, bis die Anzeige 24, wie in Figur 11 dargestellt, eine Schraubenlänge bzw. einen Näherungswert der Schraubenlänge auf der Skalierung 22 anzeigt. Die Genauigkeit des angezeigten Schraubenlängewerts ist immer von der Eingriffstiefe des Mitnehmers 28 am Schraubenkopf der Knochenschraube 29 abhängig.

In nicht dargestellten Ausführungsbeispielen dient der Messbügel als Navigator zur Findung der richtigen Schraubenposition, als Fixierung und/oder Gegenhalter beim Einschrauben der Schraube von Hand oder Maschine.

In typischen nicht dargestellten Ausführungsbeispielen weist der Bügel eine weitere Spitze auf, die es einem Operateur ermöglicht beim Andrücken der Spitze durch Haut und Gewebe an eine Knochenseite und durch Andrücken der Schrauben der Spitze auf der anderen Seite des Knochens die Schraubenlänge und -Richtung zu bestimmen.

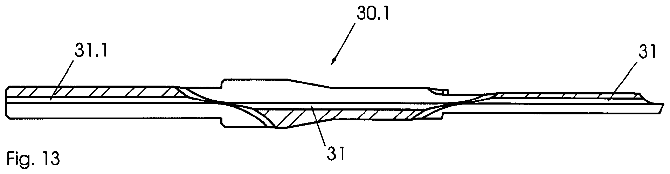

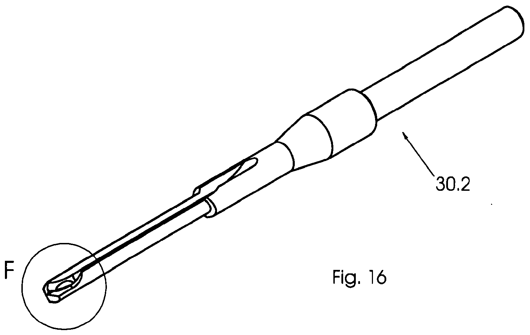

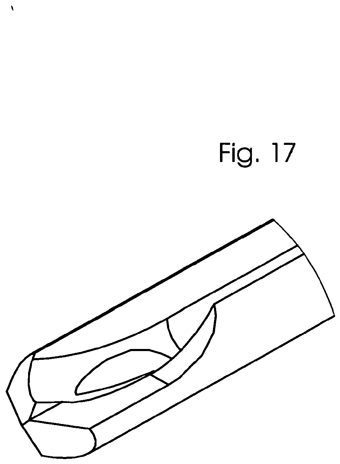

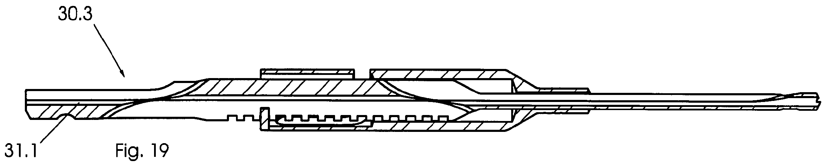

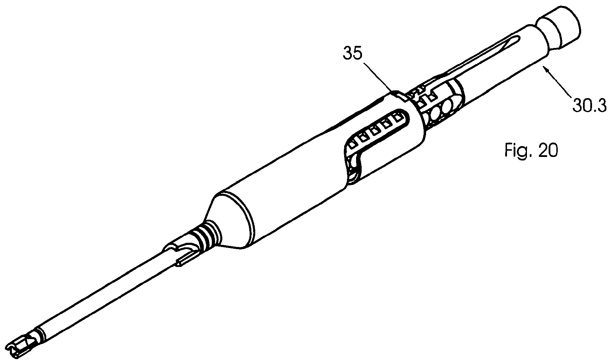

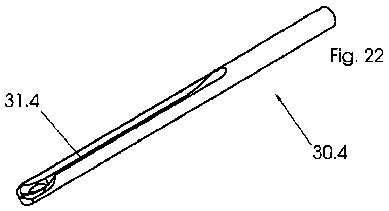

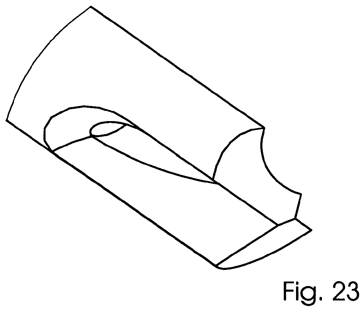

Das in den Figuren 13 bis 23 gezeigte Instrument ist bevorzugt ein Schraub-, Bohr-, Senk-, Fräs- oder Einschlag-Instrument. Vorliegend ist ein Bohrinstrument 30.1 bis 30.4 dargestellt, welches bevorzugt kanüliert (Bohrinstrument 30.1 und 30.3) und teilweise geschlitzt ausgebildet ist. In welchem Verhältnis, Abständen und/oder in welchem Winkel die Kanülierung und/oder eine Schlitzung vorgesehen ist, ist je nach Anwendung und Instrument unterschiedlich.

Bevorzugter soll mindestens ein Teil kanüliert und mindestens ein Teil von einer oder mehreren Seiten geschlitzt sein, um eine optimale Führung z.B. eines nicht näher gezeigten Bohrdrahtes zu gewährleisten.

Die Schlitze 31.1 bis 31.4 können von mehreren Seiten so angebracht sein, dass diese sich in nahezu einer Mitte bevorzugt (axial) verbinden und so eine Kanülierung für z.B. den Bohrdraht entsteht. Auch kann die Kanülierung weitere Aufgaben übernehmen wie z.B. als Kanal zum Einführen einer Sonde oder eines Ankers oder anderer Instrumente.

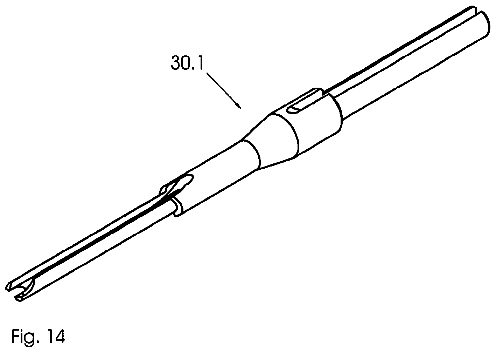

Die Figuren 14 bis 16 und 22 und 23 zeigen nicht kanülierte Bohrinstrumente 30.2 und 30.4, bei denen auf Spiralnuten am Aussenmantel verzichtet wurde. Stattdessen sind diese Bohrinstrumente 30.2 und 30.4 nur teilweise geschlitzt ausgebildet, was billiger herstellbar ist, jedoch eine vergleichbare Stabilität gewährleistet.

Bevorzugt sollen sich bei diesen Bohrinstrumenten 30.2 und 30.4 die von mehreren Seiten angebrachten Schlitze 30.2 und 30.4 an mindestens. einer Stelle verbinden oder sogar durchbrechen. So kann z.B. beim Bohren Material von einer z.B. zweiten Schneide ebenfalls in den Schlitz 30.2 und 30.4 gelangen. Vorteil ist hier, dass das Material im Schlitz schonend gesammelt wird und aus diesem einfach entnommen werden kann, um z.B. an anderer Stelle eingesetzt zu werden, oder als Probeentnahme verwendet zu werden.

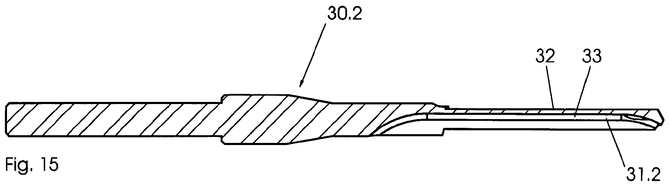

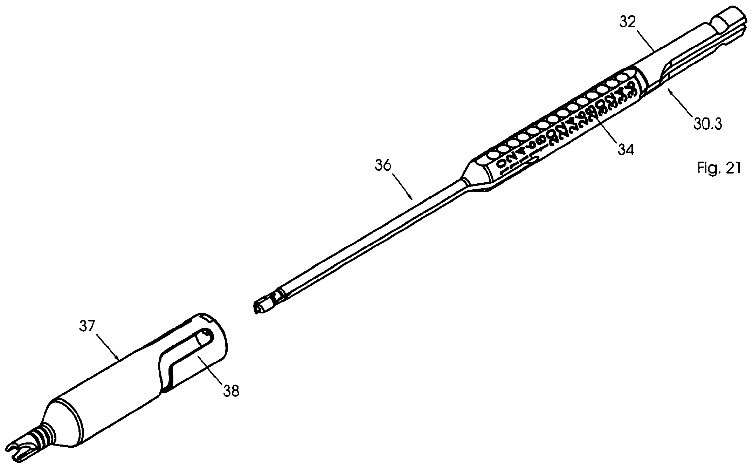

Weiterhin sind Kombinationen mit weiteren Ausführungen möglich. So können alle Instrumente 30.1 bis 30.4 entlang der Schlitze 31.1 bis 31.4 an einem Aussenmantel 32 grosszügig mit einer Markierungen 33 (siehe Figur 15) und/oder einer Skala 34, beispielsweise mit Farbmarkierungen (siehe z.B. Figur 21) und/oder Beschriftungen versehen sein. In der Regel dienen diese Markierungen 33 und/oder Skalen 34 und/oder Beschriftungen der Bestimmung einer Schraubenlänge oder einer Tiefe des in einen Knochen eingebrachten Bohrdrahtes oder Bohrers.

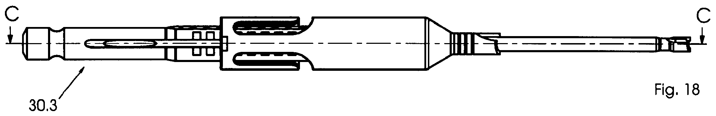

Weiterhin kann das Instrument 30.3, wie in den Figuren 18 bis 21 gezeigt, mit einem Schnellrastsystem 35 ausgestattet sein, um die Schraubenlänge einfach einstellen zu können. Hierzu ist das Bohrinstrument 30.3 mit einem Grundkörper 36 sowie einer Senkhülse 37 versehen. Die Senkhülse 37 weist ein gefedertes Druckstück 38 auf, welches, wenn die Senkhülse 37 auf den Grundkörper 36 aufgeschoben ist, ein Einrasten des Druckstückes 38 mit dem Grundkörper 36 bewirkt.

**Bezugszeichenliste**

| | | | | | |
|---|---|---|---|---|---|
| 1 | Chirurgisches Instrument | 34 | | 67 | |
| 2 | Kanülierter Schaft | 35 | | 68 | |
| 3 | Griff | 36 | | 69 | |
| 4 | Kanülierung | 37 | | 70 | |
| 5 | K-Draht | 38 | | 71 | |
| 6 | Schlitz | 39 | | 72 | |
| 7 | Skalierung | 40 | | 73 | |
| 8 | Sollbruchstelle | 41 | | 74 | |
| 9 | Knochenschraube | 42 | | 75 | |
| 10 | Ende Bohrdraht | 43 | | 76 | |
| 11 | Mitnehmer | 44 | | 77 | |
| 12 | Chirurgisches Instrument | 45 | | 78 | |
| 13 | Auswechselbarer Mitnehmer | 46 | | 79 | |
| 14 | Proximales Ende | 47 | | | |
| 15 | Knochenschraube | 48 | | | |
| 16 | Sollbruchstelle | 49 | | | |
| 17 | Mitnehmer | 50 | | | |
| 18 | Chirurgisches Instrument | 51 | | | |
| 19 | Griff | 52 | | | |
| 20 | Schaft | 53 | | | |
| 21 | Messbügel | 54 | | | |
| 22 | Skalierung | 55 | | | |
| 23 | Messspitze | 56 | | | |
| 24 | Anzeige | 57 | | | |
| 25 | Erster Haken | 58 | | | |
| 26 | Zweiter Haken | 59 | | | |
| 27 | Öffnung | 60 | | | |
| 28 | Mitnehmer | 61 | | | |
| 29 | Knochenschraube | 62 | | | |
| 30 | | 63 | | | |
| 31 | | 64 | | | |
| 32 | | 65 | | | |
| 33 | | 66 | | | |

## Patentansprüche

1. Chirurgisches Instrument (1, 12) zum Einbringen eines Implantats, umfassend:
- einen Schaft (2), wobei der Schaft (2) geeignet ist an seinem proximalen Ende das Implantat, insbesondere eine Schraube (9, 15) mit einer Kanülierung (4) aufzunehmen, wobei der Schaft (2) eine Kanülierung aufweist, und wobei der Schaft (2) an seinem proximalen Ende einen Mitnehmer (11) aufweist, geeignet mit dem Implantat in Eingriff gebracht zu werden, und
- einen Griff (3), der mit dem Schaft (2) an einem distalen Endes des Schafts (2) verbunden ist, wobei der Schaft (2) einen Schlitz (6) aufweist, und
- einen K-Draht (5), der geeignet ist in die Kanülierung des Schaftes eingebracht zu werden, und /oder ein Messbügel (21),
**dadurch gekennzeichnet,**
**dass** der Schaft (2, 20) eine Skalierung (7, 22) aufweist, wobei zur Bestimmung einer Einbringtiefe die Skalierung (7) und der K-Draht (5) dienen und/oder zur Bestimmung einer Implantatslänge die Skalierung (22) und ein Messbügel (21) dienen.

2. Chirurgisches Instrument nach Anspruch 1, **dadurch gekennzeichnet, dass** der Schaft (2, 20) an seinem proximalen Ende über eine Sollbruchstelle (8), mit einem Implantat, insbesondere einer Schraube (9) verbunden ist.

3. Verfahren zur Herstellung eines chirurgischen Instruments nach Anspruch 1 oder 2 mit der Kanülierung (4) und einem Aussenmantel, **dadurch gekennzeichnet, dass** zumindest ein Schlitz (6) in den Aussenmantel eingebildet wird, der geeignet ist die Kanülierung (4) zumindest teilweise zu bilden.

4. Verfahren nach Anspruch 3, **dadurch gekennzeichnet, dass** der Schlitz ganz oder nur teilweise über die Gesamtlänge des Aussenmantels eingebildet wird.

5. Verfahren nach Anspruch 3 oder 4, **dadurch gekennzeichnet, dass** zumindest eine Bohrung am distalen oder dem proximalen Ende des Instruments eingebracht wird.

## Claims

1. A surgical instrument (1, 12) for inserting an implant, comprising:
- a shaft (2), wherein the shaft (2) is suitable to receive the implant, in particular a screw (9, 15) with a cannulation means (4), at its proximal end, wherein the shaft (2) has a cannulation means, and
wherein the shaft (2) has at its proximal end an entraining element (11) suitable to be brought into engagement with the implant, and
- a handle (3) which is connected to the shaft (2) at a distal end of the shaft (2),
wherein the shaft (2) has a slot (6), and
- a K-wire (5) which is suitable for being introduced into the cannulation means of the shaft, and/or a measuring frame (21),
**characterised in that**
the shaft (2, 20) has a scale (7, 22), with the scale (7) and the K-wire (5) serving for determining a depth of introduction, and/or the scale (22) and a measuring frame (21) serving for determining an implant length.

2. A surgical instrument according to Claim 1, **characterised in that** the shaft (2, 20) is connected at its proximal end via a predetermined breaking point (8) to an implant, in particular a screw (9).

3. A method for producing a surgical instrument according to Claim 1 or 2 with the cannulation means (4) and an outer sheath, **characterised in that** at least one slot (6) which is suitable for forming the cannulation means (4) at least in part is formed in the outer sheath.

4. A method according to Claim 3, **characterised in that** the slot is formed entirely or only partially over the entire length of the outer sheath.

5. A method according to Claim 3 or 4, **characterised in that** at least one bore is formed at the distal or the proximal end of the instrument.

## Revendications

1. Instrument chirurgical (1, 12) pour introduire un implant, comportant:
- une tige (2), où la tige (2) convient pour recevoir, à son extrémité proximale, l'implant, en particulier une vis (9, 15) avec une canule (4), où la tige (2) présente une canule, et où la tige (2) présente, à son extrémité proximale, un organe d'entraînement (11) convenant pour être amené en prise avec l'implant, et
- une poignée (3) qui est connectée à la tige (2) à une extrémité distale de la tige (2), où la tige (2) présente une fente (6), et
- un fil en forme de K (5) qui convient pour être introduit dans la canule de la tige et/ou un étrier de mesure (21),
**caractérisé par le fait**
**que** la tige (2, 20) présente une graduation (7, 22), où pour la détermination d'une profondeur d'introduction servent la graduation (7) et le fil en forme de K (5) et/ou pour la détermination d'une longueur d'implant servent la graduation (22) et un étrier de mesure (21).

2. Instrument chirurgical selon la revendication 1, **caractérisé par le fait que** la tige (2, 20) est connectée, à son extrémité proximale, par l'intermédiaire d'un point de rupture de consigne (8), à un implant, en particulier une vis (9).

3. Procédé de fabrication d'un instrument chirurgical selon la revendication 1 ou 2 avec la canule (4) et une enveloppe extérieure, **caractérisé par le fait que** dans l'enveloppe extérieure est formée au moins une fente (6) qui convient pour former au moins partiellement la canule (4).

4. Procédé selon la revendication 3, **caractérisé par le fait que** la fente est formée entièrement ou seulement partiellement sur la longueur d'ensemble de l'enveloppe extérieure.

5. Procédé selon la revendication 3 ou 4, **caractérisé par le fait qu'**au moins un alésage est pratiqué à l'extrémité distale ou proximale de l'instrument.
